# FASCICULE DE BREVET EUROPEEN

(11) **EP 1 316 614 B1**
(45) Date de publication et mention de la délivrance du brevet: **02.07.2008**
(21) Numéro de dépôt: 02292820.4
(22) Date de dépôt: 13.11.2002
(51) Int. Cl.: C12P 19/18, C08B 30/12

(54) **Procédé continu de modification de l'amidon et de ses dérivés par enzymes de branchement**
Kontinuierliches Verfahren zur Modifizierung von Stärke und Stärkederivate mittels Verzweigungsenzyme
Continuous process for modifying starch and starch derivatives using branching enzymes

(30) Priorité: 29.11.2001 FR 0115433
(43) Date de publication de la demande: 04.06.2003
(73) Titulaire: Roquette Frères, 62136 Lestrem (FR)
(72) Inventeur: Fuertes, Patrick, 59130 Lambersart (FR); Petitjean, Carole, 59520 Marquette Lez Lille (FR)
(74) Mandataire: Boulinguiez, Didier

(56) Documents cités:
- EP-A- 0 481 903
- EP-A- 0 690 170
- WO-A-00/66633
- FR-A- 2 005 306
- US-A- 4 957 563

## Description

La présente invention est relative à un procédé de modification de l'amidon et de ses dérivés par des enzymes de branchement.

Plus particulièrement, l'invention concerne un procédé de modification de l'amidon et des dérivés de l'amidon dans lequel les enzymes de branchement sont introduites en continu dans le milieu réactionnel.

L'amidon est constitué de deux polymères, l'amylose et l'amylopectine. L'amylose est la fraction renfermant des homopolymères linéaires de glucose liés en α-1,4 et quelques points de branchement en α-1,6. L'amylopectine est quant à elle la fraction ramifiée, constituée de chaînes linéaires de glucose en α-1,4 reliées à d'autres chaînes linéaires de glucose en α-1,4 par des points de ramification en α-1,6.

L'association de ces deux homopolymères, empaquetés sous la forme de granules d'amidon très bien structurés, constitue la réserve de source carbonée de la plante.

L'amidon produit dans chaque plante est constitué d'un pourcentage variable en chacun de ses constituants amylose et amylopectine, voire même d'une distribution particulière des poids moléculaires de chacun desdits homopolymères de glucose. Ce qui explique la raison pour laquelle les divers amidons et leurs dérivés sont habituellement classés en fonction de leur origine botanique.

Les propriétés fonctionnelles des amidons et de leurs dérivés sont en outre directement fonction de leur contenu en amylose et amylopectine.

Ainsi, lorsque l'on chauffe une suspension d'amidon au delà de la température de gélatinisation, le granule d'amidon gonfle, et l'amylose se solubilise préférentiellement.

Cependant, lors du refroidissement de cette colle, notamment dans des conditions de température et de matière sèche particulières, les homopolymères de glucose rétrogradent, rapidement pour l'amylose (quelques heures), et de manière plus lente pour l'amylopectine (quelques jours).

La rétrogradation s'entend de la tendance qu'ont les macromolécules d'amylose et d'amylopectine, lors du refroidissement de ladite colle, à se réassocier entre elles, par formation de liaisons hydrogènes.

De manière pratique, elle se traduit par une opacification et une augmentation de viscosité lors du refroidissement de la colle et par la formation à froid d'une structure tridimensionnelle gélifiée.

Les spécialistes du domaine de l'utilisation des amidons et des dérivés de l'amidon en industrie alimentaire s'accordent à dire que ce phénomène de rétrogradation affecte plus particulièrement la texture des aliments, et en diminue la durée de vie.

De plus, la solubilisation de l'amylose lors de la cuisson de l'amidon, suivie d'un refroidissement, favorise sa complexation avec les lipides résiduels de l'amidon.

L'amylose se présente en effet sous une forme hélicoïdale dans laquelle les lipides peuvent s'insérer, générant alors des complexes amylose-lipides.

Ces complexes amylose-lipides conduisent également à la formation d'insolubles qui perturbent le comportement rhéologiques notamment des colles préparées à partir de ces amidons et altèrent ainsi leur état colloïdal.

Cela se traduira en papeterie par de nombreux problèmes technologiques, tant au niveau des colmatages des filtres qu'à celui de la qualité du papier.

Il est connu de rendre plus acceptables ces produits, en les préparant à partir de substances amylacées riches en amylopectine, et donc par exemple à partir des variétés waxy.

Il en résulte également bien évidemment une limitation de la formation des complexes amylose-lipides.

Cependant, la stabilité des gels et liants obtenus à partir desdits produits amylacés riches en amylopectine n'est pas suffisante pour les besoins des industries alimentaires, où il est parfois nécessaire d'avoir une durée de stockage de plusieurs mois.

Une première solution consiste à stabiliser les homopolymères de glucose, et ce grâce à des agents chimiques. Cette opération est la plupart du temps effectuée par la mise en oeuvre de réactions d'estérification ou d'éthérification. Il peut s'agir notamment de réactions d'acétylation ou d'hydroxy-propylation. En outre, pour obtenir les propriétés de texture et de viscosité souhaitées, ces réactions sont souvent associées à une réaction de réticulation.

Ces modifications confèrent alors des propriétés rhéologiques remarquables aux amidons, les rendant plus résistants aux traitements mécaniques tels le cisaillement, ou aux milieux acides. L'acétylation ou l'hydroxy-propylation confèrent en outre une bonne stabilité au stockage après cuisson, notamment à basse température.

Cependant, les produits ainsi obtenus présentent l'inconvénient d'avoir été traités chimiquement, ce qui est souvent mal perçu par les consommateurs.

Une alternative aux procédés visant à modifier chimiquement les amidons natifs de plantes mutantes, hybrides ou génétiquement modifiées, consiste à introduire *in vitro* de nouveaux points de branchement dans l'amidon.

Il s'agit alors de conduire à un remaniement des chaînes d'amylopectine ou d'amylose, plutôt que de mettre en oeuvre des réactions de stabilisation et/ou de réticulation comme indiqué précédemment.

Une technique consiste à mettre en oeuvre des enzymes purifiées de biosynthèse du glycogène et/ou de l'amidon, telles les enzymes de branchement du glycogène ou de l'amidon, responsables respectivement de la synthèse des points de ramification en α-1,6 du glycogène, ou des points de ramification en α-1,6 de l'amylopectine et des quelques points de branchement de l'amylose.

Il est ainsi décrit dans le brevet JP 60-752.95 un procédé d'obtention de substances amylacées solubles dans l'eau et de fabrication d'aliments ou de boissons en contenant, consistant à prélever la fraction soluble dans l'eau du produit issu de l'action d'une enzyme branchante sur une substance amylacée gélatinisée.

La réaction est alors effectuée en « batch », i.e. en mélangeant sans précaution particulière la substance amylacée à modifier et l'enzyme branchante.

De la même façon dans le brevet FR 2.499.588 qui couvre une enzyme branchante et la production d'aliments améliorés, une solution de substances amylacées, préparée par gélatinisation et dispersion, est d'abord soumise à l'action de l'enzyme branchante, et puis est mélangée sans traitement ultérieur ou, si nécessaire, après concentration et/ou séchage, avec les produits alimentaires.

Il est également procédé au chauffage de la substance amylacée en présence de l'enzyme branchante afin de réaliser simultanément la gélatinisation et la réaction enzymatique, et le produit résultant est ensuite incorporé dans les produits alimentaires comme souhaité.

Cependant, les enzymes de branchement mises en oeuvre dans ledit brevet ont des températures optimales d'action relativement basses (de l'ordre de 30°C pour l'enzyme extraite de *E. coli* ou de pomme de terre, et de l'ordre de 25°C pour *Bacillus megaterium*).

Il est connu en toute généralité que la température de gélatinisation de substance amylacée est inférieure à 100°C, mais une cuisson industrielle qui implique des matières sèches élevées et un temps de cuisson court, exige classiquement des températures supérieures à 100°C (comprises entre 110 et 170°C), températures bien évidemment incompatibles avec celles de fonctionnement optimales des enzymes mises en oeuvre.

La pratique préconisée dans les brevets JP 60-752.95 et FR 2.499.588 est de gélatiniser les substances amylacées dans des conditions de température qui se font au détriment d'une activité optimale des enzymes de branchement mises en oeuvre.

Cette façon de procéder ne permet donc pas de concilier le comportement rhéologique de la substance amylacée à traiter et le mode d'action des enzymes de branchement.

L'enseignement du brevet EP 690.170 relatif à un procédé de couchage et surfaçage du papier s'inscrit dans cette même logique, car il est considéré que la gélatinisation de l'amidon est le facteur essentiel, l'amidon devant être absolument gélatinisé pour permettre une action effective des enzymes de branchement.

Il est ainsi décrit de gélatiniser l'amidon en batch ou en continu, cependant que l'enzyme est introduite de manière indifférenciée, avant ou après ladite gélatinisation.

Une solution partielle a été apportée à cette difficulté de concilier optimisation de la réaction enzymatique et conditions de gélatinisation dans la demande de brevet EP 710.674, où il est décrit la mise en oeuvre de l'enzyme de branchement de pomme de terre, ou la mise en oeuvre d'une enzyme de branchement issue d'un organisme thermorésistant.

Dans le premier cas, il est recommandé d'utiliser une enzyme de branchement isolée de la pomme de terre, car sa production en grande quantité ne présente aucune difficulté majeure. L'excès d'enzyme apportée au milieu réactionnel compense donc la perte importante d'activité enzymatique que l'on va déplorer.

Cette solution n'est guère satisfaisante, car elle ne permet en aucun cas de contrôler la réaction enzymatique.

Dans le second cas, l'enzyme est recommandée parce qu'elle présente un optimum de température plus élevé que celui de l'enzyme de branchement de la pomme de terre.

Cependant, l'augmentation de la tolérance thermique des enzymes ne s'entend pas automatiquement d'une meilleure qualité des produits générés.

La mise en oeuvre de ces enzymes solutionne le problème du choc thermique lors de l'introduction de l'enzyme dans le milieu réactionnel.

Cependant, il a été constaté par la société Demanderesse l'apparition de structures de type complexes amylose-lipides dans les colles obtenues après modification de l'amidon par lesdites enzymes de branchement thermorésistantes. WO0066633 décrit des polymères solubles de glucose branchés ne contenant substantiellement pas de liaisons beta glucosidiques, qui présentent des teneurs particulières en liaisons glucosidiques alpha-1,6, une excellente stabilité en solution exprimée par leur faible tendance à la rétrogradation et une remarquable distribution des poids moléculaires dans un intervalle compris entre104 et 108 daltons.

De tout ce qui précède, il résulte qu'il y a donc un besoin non satisfait de disposer d'un procédé de modification efficace de l'amidon ou des dérivés de l'amidon par des enzymes de branchement.

Ce procédé demande notamment à ce que soient établies les conditions opératoires permettant de gérer d'une part les températures exigées pour la cuisson industrielle des amidons et des dérivés d'amidon et d'autre part, les températures correspondant à l'activité optimale des enzymes de branchement.

Ces conditions opératoires doivent permettre alors d'optimiser le fonctionnement des enzymes de branchement, ceci en limitant la formation dans le milieu réactionnel de substances insolubles, celles-ci pouvant être notamment des particules résultant de la rétrogradation de l'amidon ou des complexes issus de l'association structurée avec les lipides, ces substances insolubles gênant l'accessibilité des enzymes aux sites de branchement des chaînes glucidiques, et pouvant conduire à altérer la qualité des produits formés.

Sans être liée par une quelconque théorie, la société Demanderesse entend par « associations structurées amylose-lipides » une possible organisation de type cristallin de l'amylose et des lipides.

La société Demanderesse a eu le mérite de concilier tous ces objectifs réputés jusqu'alors difficilement conciliables en imaginant et élaborant, au prix de nombreuses recherches, un procédé de modification de l'amidon ou des dérivés de l'amidon par des enzymes de branchement qui consiste à introduire lesdites enzymes de branchement en continu dans le milieu réactionnel contenant de l'amidon ou des dérivés de l'amidon.

Le procédé de modification de l'amidon ou des dérivés de l'amidon par des enzymes de branchement conforme à l'invention consiste dans un premier temps à chauffer l'amidon ou les dérivés d'amidon de manière à être sous forme partiellement ou totalement gélatinisée.

Cette première étape du procédé conforme à l'invention assure la solubilisation de l'amidon, ou d'un dérivé de l'amidon, susceptible d'être traité par les enzymes de branchement.

Par « amidon », on entend au sens de l'invention un amidon choisi dans le groupe constitué par les amidons de maïs, de pomme de terre, de blé, de pois, de manioc et de riz.

Par « dérivés d'amidon », on entend les produits d'hydrolyse acide ou enzymatique de l'amidon, et également les produits des modifications chimiques et physiques de l'amidon de toute nature.

Dans un mode de réalisation du procédé conforme à l'invention, il est préparé un lait d'amidon d'une matière sèche comprise entre 5 et 50 %, que l'on chauffe par toute technique connue par ailleurs de l'homme du métier à une température égale ou supérieure à la température de gélatinisation de l'amidon, préférentiellement comprise entre au moins 100 et au plus 200°C, plus préférentiellement encore comprise entre au moins 110 et au plus 170°C.

Par « enzymes de branchement », on entend au sens de l'invention les enzymes de branchement choisies dans le groupe constitué par les enzymes de branchement du glycogène, les enzymes de branchement de l'amidon, les cyclomaltodextrines glucosyl transférases, les transglucosidases et les mélanges quelconques de ces enzymes.

Plus particulièrement ces enzymes de branchement sont extraites d'organismes et/ou de micro-organismes choisis dans le groupe constitué par les plantes supérieures, les levures, les bactéries et les algues unicellulaires.

Après cette étape de solubilisation totale ou partielle du lait d'amidon ou du lait de dérivés d'amidon, il est procédé conformément à l'invention, à l'introduction en continu des enzymes de branchement dans le milieu réactionnel.

Plus particulièrement, les conditions d'introduction des enzymes de branchement dans le milieu réactionnel sont réglées en temps et en températures de manière à limiter la formation des insolubles issus de la rétrogradation de l'amidon et des associations structurées amylose-lipides.

Il est donc procédé, conformément à l'invention, au refroidissement du lait d'amidon ainsi partiellement ou totalement gélatinisé, de manière à l'amener à l'optimum de température de l'enzyme de branchement choisi.

La société Demanderesse a trouvé qu'il faut ici refroidir de manière continue, rapide et contrôlée le lait d'amidon partiellement ou totalement gélatinisé jusqu'à atteindre la température de fonctionnement optimum de l'enzyme de branchement.

Par exemple, si l'on choisit l'enzyme de branchement du glycogène extraite d'*Escherichia coli* ou de microorganismes du genre *Bacillus* (*B. stearothermophilus, B. megaterium*...), ou produite à partir d'un organisme génétiquement modifiée, il faut amener la colle d'amidon à la température de l'optimum de fonctionnement de l'enzyme, i.e. comprise entre 20 et 30°C, ou comprise entre 60 et 75 °C, si l'enzyme est issue d'un microorganisme thermorésistant comme *B. stearothermophilus*.

Il sera avantageusement choisi de refroidir rapidement la solution d'amidon ou de dérivés d'amidon partiellement ou totalement gélatinisés, de sa température initiale comprise entre 100 et 200°C, dans des conditions telles qu'elles permettent d'éviter la rétrogradation de l'amidon ou la formation des associations structurées amylose-lipides, e.g. entre 1 à 15 min, comme il sera exemplifié ci-après.

Il est ensuite procédé à l'ajustement du pH de la solution, de manière à l'amener à une valeur conforme au mode de fonctionnement de ladite enzyme.

Une autre caractéristique essentielle de l'invention consiste à cette étape d'introduire en continu les enzymes de branchement.

La société Demanderesse a ainsi eu le mérite de montrer que c'est en fait par l'addition en continu des enzymes de branchement au milieu réactionnel, qu'il est possible d'optimiser le taux de modification des amidons ou dérivés d'amidon ainsi traités, et non pas en réglant les conditions de gélatinisation, comme il était recommandé de le faire dans l'état de la technique.

Par exemple, avec l'enzyme de branchement du glycogène de *E. coli* purifiée, il est avantageusement choisi d'ajouter en continu de l'enzyme diluée entre 0,5 à 15 mg/ml de protéines à un débit compris entre 90 et 600 ml/h dans un flux de la solution d'amidon ou de dérivés d'amidon de 5 à 50 % de matière sèche à un débit compris entre 0,5 et 50 l/h et refroidit entre 30 secondes et 15 minutes, comme il sera exemplifié ci-après.

A la fin de la réaction, il sera enfin procédé à la désactivation thermique de l'enzyme. Dans le cas d'une enzyme présentant un optimum de température de 30°C, il est admis qu'une élévation à la température de 70°C pendant un temps approprié inactive complètement ladite enzyme. Pour une enzyme de branchement présentant un optimum de température à 70°C, l'inactivation thermique sera effectuée à 100°C.

L'efficacité du procédé conforme à l'invention en regard des procédés classiques de modification des amidons et dérivés d'amidon est déterminée par le suivi des paramètres analytiques suivants.

La détermination du taux de liaisons α-1,6, résultant de l'action de l'enzyme de branchement, celle de la masse molaire des produits ainsi modifiés et la teneur en sucres réducteurs sont réalisées comme indiqué dans la demande de brevet WO 00/66633 dont la société Demanderesse est titulaire.

La mesure de la viscosité de la solution d'amidon ou de dérivés d'amidon ainsi traitées est effectuée en suivant le test suivant. L'analyse de viscosité consiste, en fonction des produits à analyser, à peser une masse de produit sec (3 g sec pour un amidon de maïs standard ou chimiquement modifié, 4,5 g sec pour un amidon de maïs waxy), à y ajouter 6,75 g de glycérol à 98 % de pureté dans le bol d'un « Rapid Visco Analyser » (RVA Newport), puis à compléter à 28 g avec de l'eau déminéralisée.

Il est également possible de mesurer la viscosité d'une masse de produit sec en l'absence de glycérol, en l'occurrence 7 g sec pour un amidon de maïs standard complété à 28 g avec de l'eau déminéralisée.

L'ensemble est ensuite soigneusement homogénéisé. Le profil temps / température et vitesse RVA est alors réalisé comme suit. L'échantillon est agité à 100 rpm à une température de 25°C durant 5 s, puis à 500 rpm pendant 15 s.

L'agitation est maintenue à 160 rpm durant le reste du profil. La température initiale de 25°C est maintenue durant 10 min, puis elle est augmentée à 90°C en 8 min. Cette température est maintenue 3 min, puis diminuée à 30°C en 8 min et maintenue à cette valeur de 30°C durant 5 min.

La viscosité retenue est la viscosité mesurée en centipoises en fin de profil à 30°C, à 34 min. Les bols RVA sont alors stockés à 4°C durant 7 j puis un nouveau relevé de viscosité est réalisé. Pour cela, l'échantillon est agité à 160 rpm à 30°C durant 20 min. La viscosité retenue est la moyenne de viscosité entre 15 et 20 min.

D'autres caractéristiques et avantages de l'invention apparaîtront à la lecture des exemples non limitatifs décrits ci-dessous.

### Exemple 1

Deux essais sont réalisés en batch et en continu sur de l'amidon de maïs standard avec de l'enzyme de branchement du glycogène purifiée issue d'*E*. *coli* comme indiqué ci-dessous.

Pour le procédé de modification « batch », il est préparé un lait d'amidon à 10 % de matière sèche. La solubilisation est effectuée par passage dans un cuiseur tubulaire chauffé par un fluide thermique à 145°C sous une pression de 4 à 5 bars à un débit de 40 ml/min.

1 kg de colle sont récupérés à une température proche de 100°C puis refroidis en 2 heures sous agitation pour atteindre une température de 30°C.

Le pH est ajusté à une valeur de l'ordre de 7,5 par NaOH 0,1N. 0,84 mg d'enzyme purifiée à homogénéité par gramme d'amidon sont introduits directement dans la solution à 30°C et la réaction est réalisée pendant 20 h 45. En fin de réaction, l'enzyme est désactivée par chauffage à 70°C.

Pour le procédé continu, le même lait d'amidon à 10 % de matière sèche est solubilisé par passage dans un cuiseur tubulaire chauffé par un fluide thermique à 145°C sous une pression de 4 à 5 bars à un débit de 18 ml/min.

Le refroidissement est effectué à ce même débit en 5 minutes par passage dans deux réfrigérants à - 5°C pour atteindre 30°C.

Le pH est ajusté en continu à 7,5 par NAOH 0,1 N, et l'enzyme diluée à 1,2 mg/ml est introduite en continu à un débit de 1,6 ml/min avant mélangeur en ligne.

La réaction est effectuée en réacteur thermostaté à 30°C pendant 20 h 45, et en fin de réaction, l'enzyme est désactivée par chauffage à 70 °C.

Le tableau I donne les valeurs des taux de branchement, de masse molaire, de viscosité et la teneur en sucres réducteurs des amidons standards modifiés en batch (B) et en continu (C) comparativement au témoin (A).

Pour le témoin (A), il est préparé un lait d'amidon de maïs standard à 10 % de MS. La solubilisation est effectuée par passage dans un cuiseur tubulaire chauffé par un fluide thermique à 145°C sous une pression de 4 à 5 bars à un débit de 40 ml/min. La solution est ensuite refroidie à 30°C.

**Tableau I**

| | Sucres réducteurs (%) | Taux de liaisons α-1,6 | Masse molaire MW (daltons) | Viscosité RVA (cPoises) Initiale 7j | |
|---|---|---|---|---|---|
| A | 0,15 | 3,4 | 5 10⁶ | 16100 | gel |
| B | 0,16 | 3,5 | 4,6 10⁵ | 54 | 47 |
| C | 0,16 | 5,6 | 2,9 10⁵ | 23 | 26 |

Pour les produits B et C, nous constatons la même stabilité des solutions, la même teneur en sucres réducteurs (qui confirme bien la redistribution des chaînes sans hydrolyse de l'amidon traité et l'absence d'activités amylasiques contaminantes) par rapport au témoin.

En revanche, grâce au procédé continu, le taux de branchement est significativement augmenté, et la masse molaire inférieure, avec une très faible dispersion des masses.

Les analyses en chromatographie des profils de distribution des poids moléculaires montrent bien en effet la différence entre les produits B et C.

La distribution est très resserrée et centrée sur 2.10⁵ daltons pour le produit C, alors qu'elle est plus étalée, « polydisperse » et centrée sur 3.10⁵ daltons pour le produit B.

Il est également constaté que la viscosité du produit C en solution est plus faible que celle du produit B.

Le procédé continu conforme à l'invention permet donc bien d'optimiser la réaction enzymatique.

En effet, cet exemple montre bien que la réactivité de l'enzyme de branchement est meilleure lors du procédé continu, celui-ci permettant notamment de limiter fortement la rétrogradation de l'amidon.

### Exemple 2

Deux essais sont réalisés en batch et en continu sur de l'amidon de maïs standard avec de l'enzyme de branchement du glycogène purifiée issue d'*E. coli* comme indiqué dans l'exemple 1, à la différence près que la réaction enzymatique est menée à 60°C.

Cette température de réaction est bien supérieure à la température de l'optimum de réaction de l'enzyme isolée de *E. coli*, mais elle présente l'avantage d'être plus en conformité avec les conditions industrielles classiques de mise en oeuvre d'enzyme industrielles.

Il était donc important de tester l'efficacité du procédé conforme à l'invention dans de telles conditions opératoires.

Pour le procédé de modification « batch », il est préparé un lait d'amidon à 10 % de matière sèche.

La solubilisation est effectuée par passage dans un cuiseur tubulaire chauffé par un fluide thermique à 145°C sous une pression de 4 à 5 bars à un débit de 27 ml/min.

0,5 kg de colle sont récupérés à une température proche de 100°C puis refroidis en 2 h 30 sous agitation pour atteindre la température de 60°C.

Le pH est ajusté vers 7,5 par NaOH 0,1 N et 2,2 mg d'enzyme purifiée à homogénéité par gramme d'amidon sont introduits directement dans la solution à 60°C et la réaction est réalisée pendant 19 h. En fin de réaction, l'enzyme est désactivée par chauffage à 70°C.

Pour le procédé continu, le même lait d'amidon à 10 % de matière sèche est solubilisé par passage dans un cuiseur tubulaire chauffé par un fluide thermique à 145°C sous une pression de 4 à 5 bars à un débit de 35 ml/min.

Le refroidissement est effectué à ce même débit en 6 minutes pour atteindre 60°C. Le pH est ajusté en continu à 7,5 par NAOH 0,1 N, et l'enzyme diluée à 3,1 mg/ml est introduite en continu à un débit de 2,5 ml/min avant mélangeur en ligne.

La réaction est effectuée en réacteur thermostaté à 60°C pendant 22 h 30, et en fin de réaction, l'enzyme est désactivée par chauffage à 70 °C.

Le tableau II donne les valeurs des taux de branchement, de masse molaire, de viscosité et la teneur en sucres réducteurs des amidons standards modifiés en batch (E) et en continu (F) comparativement au témoin (D).

Pour le témoin (D), il est préparé un lait d'amidon de maïs standard à 10 % de MS. La solubilisation est effectuée par passage dans un cuiseur tubulaire chauffé par un fluide thermique à 145°C sous une pression de 4 à 5 bars à un débit de 28 ml/min. La solution est ensuite refroidie à 60°C.

**Tableau II**

| | Sucres réducteurs (%) | Taux de liaisons α-1,6 | Masse molaire MW (daltons) | Viscosité RVA (cPoises) Initiale 7j | |
|---|---|---|---|---|---|
| D | 0,1 | 3,4 | > 5.10⁶ | 17003 | gel |
| E | 0,1 | 3,2 | > 5.10⁶ | 11953 | gel |
| F | 0,1 | 3,8 | > 5.10⁶ | 3675 | gel |

L'ajout en continu de l'enzyme, même à une température peu tolérée par l'enzyme de branchement, permet d'améliorer le taux de branchement de l'amidon ainsi obtenu, et de lui conférer des propriétés physico-chimiques nouvelles, comme l'illustrent par ailleurs les résultats obtenus de viscosité de la solution.

Cet exemple illustre encore l'avantage du procédé avec ajout en continu de l'enzyme.

En effet, même à une température qui limite les phénomènes de rétrogradation de l'amidon de maïs standard, l'ajout en continu de l'enzyme de branchement permet d'obtenir un produit dont les caractéristiques sont améliorées par rapport au produit réalisé avec un procédé batch.

### Exemple 3

Un essai est réalisé avec un maïs waxy, même s'il est entendu qu'un amidon de cette qualité présente une plus faible tendance à la rétrogradation.

Cependant, il est également admis que notamment dans les applications papetières, par exemple après un traitement oxydant, il est difficile de maintenir la stabilité des préparations préparées à partir dudit amidon waxy.

Il était donc intéressant de tenter d'obtenir, après modification en continu, un amidon waxy encore plus transformé que lors d'un procédé de modification batch.

Pour le procédé de modification « batch », il est préparé un lait d'amidon waxy à 15 % de matière sèche.

La solubilisation est effectuée par passage dans un cuiseur tubulaire chauffé par un fluide thermique à 145°C sous une pression de 4 à 5 bars à un débit de 25 ml/min.

1 kg de colle sont récupérés à une température proche de 100°C puis refroidis en 4 h 15 sous agitation pour atteindre la température de 30°C.

Le pH est ajusté vers 7,5 par NaOH 0,1N et 2,1 mg d'enzyme purifiée à homogénéité par gramme d'amidon sont introduits directement dans la solution à 30°C et le mélange est mis à réagir pendant 20 h. En fin de réaction, l'enzyme est désactivée par chauffage à 70°C.

Pour le procédé continu, le même lait d'amidon à 15 % de matière sèche est solubilisé par passage dans un cuiseur tubulaire chauffé par un fluide thermique à 145°C sous une pression de 4 à 5 bars à un débit de 25 ml/min.

Le refroidissement est effectué à ce même débit en 5 à 10 minutes pour atteindre 30°C. Le pH est ajusté en continu à 7,5 par NAOH 0,1 N, et l'enzyme diluée à 3,1 mg/ml est introduite en continu à un débit de 2,5 ml/min avant mélangeur en ligne.

La réaction est effectuée en réacteur thermostaté à 30°C pendant 22 h 30, et en fin de réaction, l'enzyme est désactivée par chauffage à 70 °C.

Le tableau III donne les valeurs des taux de branchement, de masse molaire, de viscosité et la teneur en sucres réducteurs des amidons waxy modifiés en batch (H) et en continu (I) comparativement au témoin (G).

Pour le témoin (G), il est préparé un lait d'amidon de maïs waxy à 15 % de MS. La solubilisation est effectuée par passage dans un cuiseur tubulaire chauffé par un fluide thermique à 145°C sous une pression de 4 à 5 bars à un débit de 25 ml/min. La solution est ensuite refroidie à 30°C.

**Tableau III**

| | Sucres réducteurs (%) | Taux de liaisons α-1,6 | Masse molaire MW (daltons) | Viscosité RVA (cPoises) Initiale 7j | |
|---|---|---|---|---|---|
| G | 0,03 | 4,3 | > 5.10⁶ | 4120 | 8096 |
| H | 0,04 | 6,5 | 2,8 10⁵ | 50 | 60 |
| I | 0,05 | 7,8 | 2,5 10⁵ | 54 | 65 |

Le traitement selon l'invention de l'amidon waxy par de l'enzyme de branchement en continu permet donc, par rapport au procédé batch d'augmenter de manière significative le taux de branchement et de générer des produits présentant encore une bonne stabilité dans le temps et un comportement rhéologique satisfaisant.

### Exemple 4

Un essai est réalisé avec un maïs waxy, comme dans l'exemple 3, mais il est procédé à un traitement par l'enzyme de branchement à 60°C.

Pour le procédé de modification « batch », il est préparé un lait d'amidon waxy à 15 % de matière sèche.

La solubilisation est effectuée par passage dans un cuiseur tubulaire chauffé par un fluide thermique à 145°C sous une pression de 4 à 5 bars à un débit de 22 ml/min.

0,5 kg de colle sont récupérés à une température proche de 100°C puis refroidis en 1 h 30 sous agitation pour atteindre la température de 60°C.

Le pH est ajusté vers 7,5 par NaOH 0,1N et 2,2 mg d'enzyme purifiée à homogénéité par gramme d'amidon sont introduits directement dans la solution à 60°C et la réaction est réalisée pendant 19 h. En fin de réaction, l'enzyme est désactivée par chauffage à 70°C.

Pour le procédé continu, le même lait d'amidon à 15 % de matière sèche est solubilisé par passage dans un cuiseur tubulaire chauffé par un fluide thermique à 145°C sous une pression de 4 à 5 bars à un débit de 25 ml/min.

Le refroidissement est effectué à ce même débit en 8 minutes pour atteindre 60°C. Le pH est ajusté en continu à 7,5 par NAOH 0,1 N, et l'enzyme diluée à 3,1 mg/ml est introduite en continu à un débit de 2,9 ml/min avant mélangeur en ligne.

La réaction est effectuée en réacteur thermostaté à 60°C pendant 22 h 30, et en fin de réaction, l'enzyme est désactivée par chauffage à 70 °C.

Le tableau IV donne les valeurs des taux de branchement, de masse molaire, de viscosité et la teneur en sucres réducteurs des amidons waxy modifiés en batch (K) et en continu (L) comparativement au témoin (J).

Pour le témoin (J), il est préparé un lait d'amidon de maïs waxy à 15 % de MS. La solubilisation est effectuée par passage dans un cuiseur tubulaire chauffé par un fluide thermique à 145°C sous une pression de 4 à 5 bars à un débit de 22 ml/min. La solution est ensuite refroidie à 60°C.

**Tableau IV**

| | Sucres réducteurs (%) | Taux de liaisons α-1,6 | Masse molaire MW (daltons) | Viscosité RVA (cPoises) Initiale 7j | |
|---|---|---|---|---|---|
| J | 0,03 | 4,7 | > 5 10⁶ | 2589 | 6511 |
| K | 0,03 | 4,8 | > 5 10⁶ | 2303 | gel |
| L | 0,04 | 6,3 | > 5 10⁶ | 2105 | 3382 |

Le traitement selon l'invention de l'amidon waxy par de l'enzyme de branchement en continu permet donc, par rapport au procédé batch d'augmenter de manière significative le taux de branchement et de générer des produits présentant encore une bonne stabilité dans le temps et un comportement rhéologique tout à fait conforme aux modifications effectuées en procédé batch.

### Exemple 5

Deux essais sont réalisés en batch et en continu sur de l'amidon de maïs standard avec de l'enzyme de branchement du glycogène purifiée issue d'*B. stearothermophilus* comme indiqué ci-dessous.

Pour le procédé de modification « batch », il est préparé un lait d'amidon à 10 % de matière sèche.

La solubilisation est effectuée par passage dans un cuiseur tubulaire chauffé par un fluide thermique à 145°C sous une pression de 4 à 5 bars à un débit de 32 ml/min.

1,8 kg de colle sont récupérés à une température proche de 100°C puis refroidis en 1 h 30 sous agitation pour atteindre la température de 70°C.

Le pH est ajusté vers 6,5 par NaOH 0,1 N et 0,026 mg d'enzyme purifiée à homogénéité par gramme d'amidon sont introduits directement dans la solution à 70°C et la réaction est réalisée pendant 23 h. En fin de réaction, l'enzyme est désactivée par chauffage à 100°C.

Pour le procédé continu, le même lait d'amidon à 10 % de matière sèche est solubilisé par passage dans un cuiseur tubulaire chauffé par un fluide thermique à 145°C sous une pression de 4 à 5 bars à un débit de 32 ml/min.

Le refroidissement est effectué à ce même débit en 5-10 minutes pour atteindre 70°C. Le pH est ajusté en continu à 6,5 par NAOH 0,1 N, et l'enzyme diluée à 0,026 mg/ml est introduite en continu à un débit de 3,3 ml/min avant mélangeur en ligne.

La réaction est effectuée en réacteur thermostaté à 70°C pendant 23 h, et en fin de réaction, l'enzyme est désactivée par chauffage à 100 °C.

Le tableau V donne les valeurs des taux de branchement, de masse molaire, de viscosité et la teneur en sucres réducteurs des amidons standards modifiés en batch (N) et en continu (O) comparativement au témoin (M).

Pour le témoin (M), il est préparé un lait d'amidon de maïs standard à 10 % de MS. La solubilisation est effectuée par passage dans un cuiseur tubulaire chauffé par un fluide thermique à 145°C sous une pression de 4 à 5 bars à un débit de 32 ml/min. La solution est ensuite refroidie à 70°C.

**Tableau V**

| | Sucres réducteurs (%) | Taux de liaisons α-1,6 | Masse molaire MW (daltons) | Viscosité RVA (cPoises) à 25 % de MS Initiale 7j | |
|---|---|---|---|---|---|
| M | 0,1 | 3,4 | > 5.10⁶ | > 24000 | gel |
| N | 0,13 | 6,8 | 1,6 10⁵ | 153 | 150 |
| O | 0,12 | 6,5 | 1,8 10⁵ | 99 | 106 |

Les résultats ne montrent apparemment pas de différences significatives entre le traitement en batch et le traitement en continu de l'amidon de maïs standard par l'enzyme de branchement thermorésistante issue de *B. stearothermophilus*.

Cependant, les colles ainsi obtenues, recueillies après désactivation de l'enzyme à 100°C et centrifugées, présentent des précipités en quantités totalement différentes entre le traitement en batch et le traitement en continu.

Ces précipités correspondent à des structures de type de celles obtenues par complexation des lipides à la fraction amylose de l'amidon traité.

Dans le procédé de modification de l'amidon en batch, ces précipités atteignent de l'ordre de 12,9 % en poids de la colle, tandis que le traitement en continu permet de limiter ces précipités d'un facteur de l'ordre de 4.

Le procédé de modification de l'amidon en continu conforme à l'invention est donc particulièrement bien adapté à la préparation de colles d'excellente qualité.

## Revendications

1. Procédé de modification de l'amidon ou des dérivés de l'amidon par des enzymes de branchement, **caractérisé en ce qu'**il consiste à :
1) chauffer l'amidon ou les dérivés d'amidon de manière à obtenir un lait d'amidon partiellement ou totalement gélatinisée,
2) refroidir entre 1 à 15 min le lait d'amidon ainsi obtenu de manière à l'amener à l'optimum de température de l'enzyme de branchement,
3) introduire lesdites enzymes de branchement en continu dans le milieu réactionnel contenant de l'amidon ou des dérivés de l'amidon.

2. Procédé selon la revendication 1, **caractérisé en ce que** les conditions d'introduction en continu des enzymes de branchement dans le milieu réactionnel sont réglées en temps et en températures.

3. Procédé selon l'une ou l'autre des revendications 1 à 2, **caractérisé en ce que** les enzymes de branchement sont extraites d'organismes et/ou de micro-organismes choisis dans le groupe constitué par les plantes supérieures, les levures, les bactéries et les algues unicellulaires.

4. Procédé selon l'une quelconque des revendications 1 à 3, **caractérisé en ce que** les enzymes de branchement sont choisies dans le groupe constitué par les enzymes de branchement du glycogène, les enzymes de branchement de l'amidon, les cyclomaltodextrines glucosyl transférases, les transglucosidases et les mélanges quelconques de ces enzymes.

5. Procédé selon l'une quelconque des revendications 1 à 4, **caractérisé en ce que** l'amidon est choisi dans le groupe constitué par les amidons de mais, de pomme de terre, de blé, de pois, de manioc et de riz.

6. Procédé selon l'une quelconque des revendications 1 à 5, **caractérisé en ce que** les dérivés de l'amidon sont choisis dans le groupe constitué par les produits d'hydrolyse acide ou enzymatique de l'amidon, et également les produits des modifications chimiques et physiques de l'amidon de toute nature.

## Claims

1. Process for modifying starch or starch derivatives by branching enzymes, **characterized in that** it consists in:
1) heating the starch or starch derivatives so as to obtain a starch milk partially or completely gelatinized,
2) cooling within 1 to 15 minutes the starch milk obtained so as to bring it to the temperature optimum for the branching enzyme,
3) continuously introducing said branching enzymes into the reaction medium containing starch or starch derivatives.

2. Process according to Claim 1, **characterized in that** the conditions for continuously introducing the branching enzymes into the reaction medium are set with respect to the time and the temperatures.

3. Process according to Claim 1 or 2, **characterized in that** the branching enzymes are extracted from organisms and/or microorganisms selected from the group consisting of higher plants, yeasts, bacteria and unicellular algae.

4. Process according to any one of Claims 1 to 3, **characterized in that** the branching enzymes are selected from the group consisting of glycogen branching enzymes, starch branching enzymes, cyclomaltodextrin glucosyl transferases, transglucosidases and any mixtures of these enzymes.

5. Process according to any one of Claims 1 to 4, **characterized in that** the starch is selected from the group consisting of maize, potato, wheat, pea, cassava and rice starches.

6. Process according to any one of Claims 1 to 5, **characterized in that** the starch derivatives are selected from the group consisting of the products of acid or enzymatic hydrolysis of starch, and also the products of the chemical and physical modifications of starch of any type.

## Patentansprüche

1. Verfahren zur Modifizierung von Stärke oder Stärkederivaten durch Verzweigungsenzyme, **dadurch gekennzeichnet, dass** es besteht aus:
1) Erhitzen von Stärke oder Stärkederivaten auf eine Art, um Stärkemilch zu erhalten, die teilweise oder vollständig gelatiniert ist,
2) Abkühlen für 1 bis 15 Minuten der so erhaltenen Stärkemilch, um sie auf die für das Verzweigungsenzym optimale Temperatur zu bringen,
3) kontinuierlichem Einbringen der Verzweigungsenzyme in das die Stärke oder die Stärkederivate enthaltende Reaktionsmedium.

2. Verfahren gemäß Anspruch 1, **dadurch gekennzeichnet, dass** die Bedingungen für das kontinuierliche Einbringen der Verzweigungsenzyme in das Reaktionsmedium zeitlich und bezüglich der Temperatur geregelt sind.

3. Verfahren gemäß einem der Ansprüche 1 oder 2, **dadurch gekennzeichnet, dass** die Verzweigungsenzyme von Organismen und/oder Mikroorganismen gewählt aus der Gruppe bestehend aus den höheren Pflanzen, den Hefen, den Bakterien und den einzelligen Algen extrahiert sind.

4. Verfahren gemäß einem der Ansprüche 1 bis 3, **dadurch gekennzeichnet, dass** die Verzweigungsenzyme gewählt sind aus der Gruppe bestehend aus den Verzweigungsenzymen von Glycogen, den Verzweigungsenzymen von Stärke, den Glucosylcyclomaltodextrintransferasen, den Transglucosidasen und beliebigen Gemischen dieser Enzyme.

5. Verfahren gemäß einem der Ansprüche 1 bis 4, **dadurch gekennzeichnet, dass** die Stärke gewählt ist aus der Gruppe bestehend aus den Stärken von Mais, Kartoffel, Weizen, Erbse, Maniok und Reis.

6. Verfahren gemäß einem der Ansprüche 1 bis 5, **dadurch gekennzeichnet, dass** die Stärkederivate gewählt sind aus der Gruppe bestehend aus den Produkten der sauren oder enzymatischen Hydrolyse von Stärke und gleichermaßen aus den Produkten der chemischen und physikalischen Modifikationen von Stärke jeder Art.
